# EUROPEAN PATENT SPECIFICATION

(11) **EP 3 941 550 B1**
(45) Date of publication and mention of the grant of the patent: **17.12.2025**
(21) Application number: 20714696.0
(22) Date of filing: 16.03.2020
(51) Int. Cl.: A61M 1/36

(54) **BIOMEDICAL CONNECTING DEVICE FOR MEASURING BLOOD PRESSURE**
BIOMEDISCHE VERBINDUNGSVORRICHTUNG ZUR BLUTDRUCKMESSUNG
DISPOSITIF CONNECTEUR BIOMÉDICAL POUR MÉSURER LA PRESSION SANGUINE

(30) Priority: 22.03.2019 IT 201900004193
(43) Date of publication of application: 26.01.2022
(73) Proprietor: Eurosets S.r.l., 41036 Medolla (MO) (IT)
(72) Inventor: PETRALIA, Antonio, 41036 Medolla (MO) (IT); GHELLI, Nicola, 41036 Medolla (MO) (IT); FONTANILI, Paolo, 41036 Medolla (MO) (IT)
(74) Representative: Brunacci, Marco
(86) International application number: PCT/IB2020/052376
(87) International publication number: WO 2020/194118

(56) References cited:
- EP-A1- 0 809 966
- IT-A1- MO20 100 318
- US-B1- 6 526 357

## Description

### Technical Field

The present invention relates to a biomedical joint device for the measurement of physical quantities, wherein the physical quantities are pressure and/or temperature.

### Background Art

In the biomedical sector, the use of devices for the treatment of body fluids, such as e.g. oxygenators, centrifugal pumps, etc., generally used in extracorporeal circulation treatments applied to a patient, is well known.

During the operation of these devices there is the need to monitor the pressure of the treated body fluid at the inlet and/or at the outlet of the devices themselves, in order to check the correct operation thereof.

For example, in the case of the oxygenator, a difference in pressure between inlet and outlet, above a predefined value, may indicate the occlusion of the oxygenator itself.

In order to detect the blood pressure at the inlet and/or outlet of such a device, a joint communicating with a relevant inlet and/or outlet duct is generally used, to which a pipe is connected, along which a deformable membrane is arranged and adapted to send a signal corresponding to the value of the blood pressure contained in the pipe itself.

The use of these pipes can however lead to the coagulation of blood contained therein, especially in emergency situations where the patient is not previously treated with anticoagulants.

As a result, the aforementioned pipes need to undergo frequent washing operations during the patient's treatment, e.g. by means of physiological saline solution, in order to remove any clots.

The aforementioned operations, therefore, entail a considerable complexity in the execution of the processes and a consequent lengthening of the treatment times.

US 6526357 B1 discloses a connector device for measuring blood pressure in an extracorporeal blood-circuit of the known type, EP 0809966 A1 discloses a device for measuring blood parameters in an extracorporeal blood-circuit of the known type and IT MO20100318 A1 discloses a biomedical connector comprising a tubular connecting element with two ports.

### Description of the Invention

The main aim of the present invention is to devise a biomedical joint device for the measurement of pressure and/or temperature of blood comprising: - one tubular joint element provided with at least one connecting channel defining a first port, connectable to a biomedical device for the treatment of blood, and a second port opposite to said first port; - sensor means, positioned adjacent to said second port and communicating with said connecting channel, adapted to detect the pressure and/or temperature of blood, said sensor means comprising at least one pressure sensor adapted to detect the pressure of the blood and/or at least one temperature sensor adapted to detect the temperature of the blood;- transmission means for transmitting said pressure and/or temperature of the blood to said sensor means where said transmission means are housed in said connecting channel and are adapted to interact with said sensor means, said connecting channel being engaged at least partially by said transmission means, where said transmission means comprise at least one pressure transmission element of the elastically deformable type and arranged inside said connecting channel so as to cover at least said second port and/or at least one temperature transmission element of the blood to said temperature sensor, characterized by the fact that it comprises at least one supporting element of said sensor means associated with said joint element, to close said second port, where said joint element is locked together with said supporting element at the area where said sensor means are defined, said sensor means being arranged at the point where said second port is located, thus resulting in communication with said connecting channel.

This biomedical device eliminates the risks of the known solutions used today.

Another object of the present invention is to devise a biomedical joint device for the measurement of physical quantities that allows a practical and safe use even in the emergency cases where the patient is not previously treated with anticoagulants.

Within this aim, a further object of the present invention is to eliminate the risk of clots and, at the same time, to simplify the measuring operations.

Another object of the present invention is to devise a biomedical joint device for the measurement of physical quantities that allows overcoming the aforementioned drawbacks of the prior art within a simple, rational, easy, effective to use and affordable solution.

The objects set out above are achieved by the present biomedical joint device for the measurement of physical quantities with the characteristics of claim 1.

### Brief Description of the Drawings

Other characteristics and advantages of the present invention will be more evident from the description of a preferred embodiment of a biomedical joint device for the measurement of physical quantities illustrated by way of an indicative example in the attached tables of drawings in which:

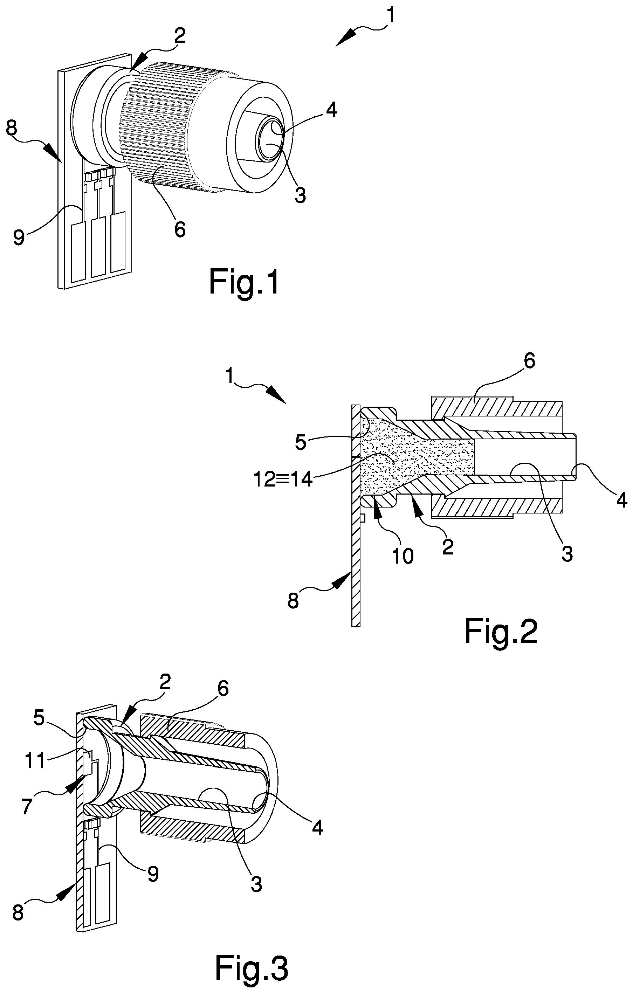

Figure 1 is an axonometric view of the biomedical joint device for the measurement of physical quantities according to the invention;
Figure 2 is a side sectional view of the invention;
Figure 3 is an axonometric view of a lateral section of the invention, in an embodiment;
Figure 4 is an exploded view of the invention, in an embodiment;
Figure 5 is a view of a further embodiment of the present invention.

### Embodiments of the Invention

With particular reference to these figures, reference numeral 1 globally designates a biomedical joint device for the measurement of physical quantities.

The biomedical joint device for the measurement of physical quantities 1 comprises at least one tubular joint element 2 provided with at least one connecting channel 3 defining a first port 4, connectable to a biomedical device for the treatment of a body fluid and adapted to allow the latter to enter the connecting channel 3, and a second port 5 opposite to the first port 4.

Advantageously, the joint element 2 comprises a connector of the Luer-lock type defined at the first port 4, which makes it possible to connect the biomedical joint device for the measurement of physical quantities 1 to any biomedical device standardised according to universal standards.

In more detail, the joint element 2 can be a Luer-lock connector, with a fixed ring nut, or comprising locking means 6 of the type of a rotating collar, to allow easy coupling to the relevant biomedical device.

According to the invention, the device 1 also comprises sensor means 7 adapted to detect at least one physical quantity characteristic of the body fluid, and at least one supporting element 8 of the aforementioned sensor means 7, associated with the joint element 2 and arranged so as to close the second port 5.

Preferably, the supporting element 8 has a substantially sheet-like shape and is made of a semiconductor material, particularly a ceramic material, with a substantially rectangular shape, on which, at one end the sensor means 7 are positioned.

The supporting element 8 comprises at least one integrated electric circuit 9, operationally connected to the sensor means 7, and is adapted to transfer the electrical pulses generated by the sensor means themselves to a processing unit through electrical connecting means 16.

The connecting means 16 are associated with the electric circuit 9 and are e.g. of the type of electrical wires or optical fibres.

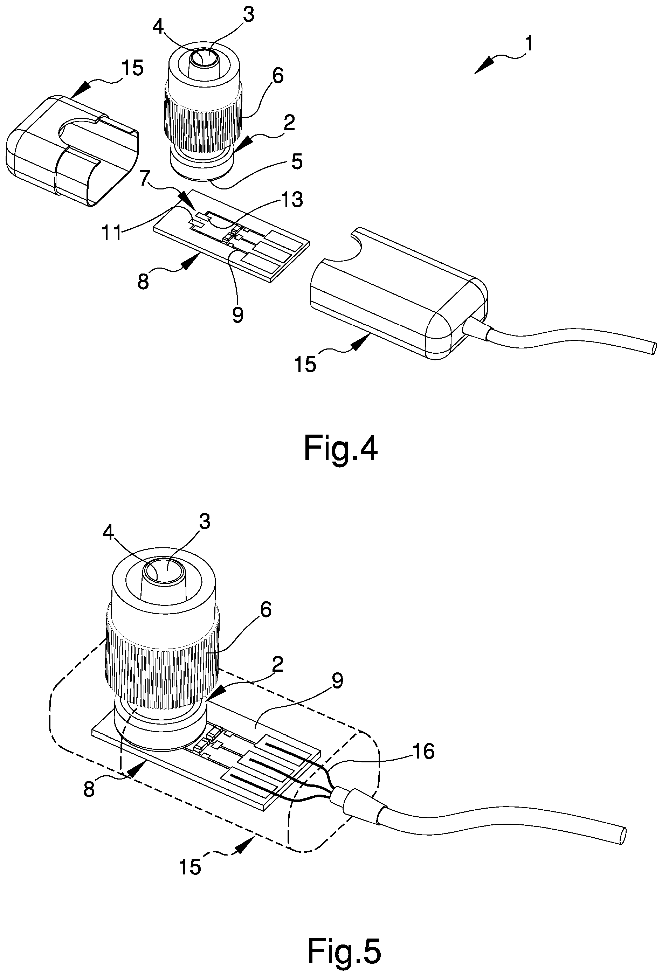

In the embodiment shown in Figure 5, the connecting means 16 are of the fixed type, i.e. they are connected to the electric circuit 9 by means of electrical soldering.

In an alternative embodiment, not shown in the figures, the electrical connecting means 16 are of the removable type, i.e. the connection to the electric circuit 9 can be made via a special connector, e.g. of the type of a USB port or the like.

In this case, the connecting means 16 can be removed from the electric circuit 9 and, therefore, from the supporting element 8 so that it can be applied to another supporting element 8 for later use.

Specifically, the joint element 2 is locked together with the supporting element 8 at the area where the sensor means 7 are defined; these are, therefore, arranged at the point where the second port 5 is located, thus resulting in communication with the connecting channel 3.

The device 1 also conveniently comprises transmission means 10 for transmitting the characteristic physical quantity to be detected to the sensor means 7.

The transmission means 10 are housed in the connecting channel 3 and are adapted to interact with the sensor means 7; more specifically, the connecting channel 3 is at least partially engaged by them.

The transmission means 10, therefore, may only partially occupy the connecting channel 3, thus covering the second port 5, or can occupy the entire volume.

The transmission means 10 also have the function of avoiding direct interaction of the body fluid with the sensor means 7, protecting the latter from wear and tear and at the same time avoiding contamination of the body fluid by any impurities.

Advantageously, the transmission means 10 are of the type of a biocompatible gel, which also allows reducing the risks to the patient's health related to any possible contamination of the body fluid by the transmission means themselves.

It should be pointed out that the particular conformation of the device 1 allows a rapid interaction between the body fluid, entering the connecting channel 3, and the sensor means 7, thus allowing an immediate detection of the characteristic physical quantities to be measured and avoiding the excessive stationing of the body fluid inside the connecting channel 3.

Conveniently, the sensor means 7 comprise a pressure sensor 11, adapted to detect the pressure of the body fluid, e.g. of the type of a pressure transducer, and the transmission means 10 comprise at least one pressure transmission element 12 of an elastically deformable type and arranged inside the connecting channel 3 so as to cover at least the second port 5.

In particular, the pressure transmission element 12 is made of an elastically deformable material adapted to compress or stretch depending on the pressure of the body fluid, thus exerting a relevant force on the pressure sensor 11.

In a particular embodiment of the present invention, the sensor means 7 comprise at least one temperature sensor 13 adapted to detect the temperature of the body fluid and the transmission means 10 comprise at least one temperature transmission element 14 of the body fluid to the temperature sensor 13.

More specifically, the temperature transmission element 14 is of the type of a thermally conductive material adapted to transmit heat to the temperature sensor 13.

The device 1 can only comprise one of either the pressure sensor 11 or the temperature sensor 13, or it can comprise both.

In the latter case, the pressure transmission element 12 and the temperature transmission element 14 can be separate, or coincide in a single element made of an elastically deformable and thermally conductive material.

Preferably, the device 1 is also provided with containment means 15 of the supporting element 8, adapted to protect the supporting element itself and, therefore, the electric circuit 9 and the connecting means 16 from external agents that may jeopardize the operation thereof.

If the connecting means 16 are of the removable type, they may be pulled out from the containment means 15 to enable them to be reused.

The operation of the device 1 is as follows.

The device 1 is connected, through the joint element 2, to a branch of a biomedical device for the treatment of a body fluid, such as e.g. an oxygenator or a centrifugal pump.

Once the body fluid reaches the branch, it enters the connecting channel 3 and, interacting with the transmission means 10, allows the sensor means 7 to detect the characteristic physical quantities to be measured.

Using the connecting means 16, the electrical pulses are transferred from the electric circuit 9 to the processing unit to enable the measured physical quantities to be read.

At the end of the treatment, the device 1 can be removed from the relevant biomedical device so that it can be easily cleaned and sterilized for later use.

It has in practice been found that the described invention achieves the intended objects and in particular the fact is underlined that the biomedical joint device for the measurement of physical quantities according to the invention allows detecting the characteristic physical quantities of a body fluid in an easy and precise way, thus eliminating at the same time the risks connected to the stationing of the body fluid itself inside the pipes used in the known devices.

In fact, the particular conformation of the biomedical joint device for the measurement of physical quantities and the presence of the transmission means for the transmission of the characteristic physical quantity to be detected allows reducing to a minimum the quantity of body fluid contained inside the connecting channel.

This also makes it possible to avoid complex and frequent washing operations, making the measurement of the characteristic physical quantity of interest significantly easier than the devices known today.

## Claims

1. Biomedical joint device for the measurement of pressure and/or temperature of blood (1) comprising:
- one tubular joint element (2) provided with at least one connecting channel (3) defining a first port (4), connectable to a biomedical device for the treatment of blood, and a second port (5) opposite to said first port (4);
- sensor means (7), positioned adjacent to said second port (5) and communicating with said connecting channel (3), adapted to detect the pressure and/or temperature of blood, said sensor means (7) comprising at least one pressure sensor (11) adapted to detect the pressure of the blood and/or at least one temperature sensor (13) adapted to detect the temperature of the blood;
- transmission means (10) for transmitting said pressure and/or temperature of the blood to said sensor means (7) where said transmission means (10) are housed in said connecting channel (3) and are adapted to interact with said sensor means (7), said connecting channel (3) being engaged at least partially by said transmission means (10), where said transmission means (10) comprise at least one pressure transmission element (12) of the elastically deformable type and arranged inside said connecting channel (3) so as to cover at least said second port (5) and/or at least one temperature transmission element (14) of the blood to said temperature sensor (13).
**characterized by** the fact that it comprises
- at least one supporting element (8) of said sensor means (7) associated with said joint element (2), to close said second port (5), where said joint element (2) is locked together with said supporting element (8) at the area where said sensor means (7) are defined, said sensor means (7) being arranged at the point where said second port (5) is located, thus resulting in communication with said connecting channel (3).

2. Device (1) according to claim 1, **characterized by** the fact that said joint element (2) comprises a connector of the Luer-lock type defined at said first port (4).

3. Device (1) according to claim 2, **characterized by** the fact that said joint element (2) comprises locking means (6) to said biomedical device, of the type of a rotating collar.

4. Device (1) according to one or more of the preceding claims, **characterized by** the fact that said pressure sensor (11) is of the type of a pressure transducer.

5. Device (1) according to one or more of the preceding claims, **characterized by** the fact that said transmission means (10) are of the type of a biocompatible gel.

6. Device (1) according to one or more of the preceding claims, **characterized by** the fact that said supporting element (8) comprises at least one integrated electric circuit (9), operationally connected to said sensor means (7), said electric circuit (9) being adapted to transfer the electrical pulses generated by said sensor means (7) to a processing unit.

7. Device (1) according to one or more of the preceding claims, **characterized by** the fact that it comprises containment means (15) of said supporting element (8).

8. Device (1) according to one or more of the preceding claims, **characterized by** the fact that it comprises connecting means (16) of said electric circuit (9) to a processing unit of the detected physical quantities.

9. Device (1) according to claim 8, **characterized by** the fact that said connecting means (16) are associated in a removable manner with said electric circuit (9).

## Patentansprüche

1. Biomedizinische Verbindungsvorrichtung zur Messung des Drucks und/oder der Temperatur von Blut (1), umfassend:
- ein rohrförmiges Verbindungselement (2), das mit mindestens einem Verbindungskanal (3) versehen ist, der eine erste Schnittstelle (4) definiert, die mit einer biomedizinischen Vorrichtung für die Behandlung von Blut verbunden werden kann, und eine zweite Schnittstelle (5), die der ersten Schnittstelle (4) gegenüberliegt;
- Sensormittel (7), die angrenzend an die zweite Schnittstelle (5) angeordnet sind und mit dem Verbindungskanal (3) in Verbindung stehen, die geeignet sind, den Druck und/oder die Temperatur von Blut zu erfassen, wobei die Sensormittel (7) mindestens einen Drucksensor (11) umfassen, der dazu ausgebildet ist, den Druck des Blutes zu erfassen, und/oder mindestens einen Temperatursensor (13), der dazu ausgebildet ist, die Temperatur des Blutes zu erfassen;
- Übertragungsmittel (10) zum Übertragen des Drucks und/oder der Temperatur des Bluts an die Sensormittel (7), wobei die Übertragungsmittel (10) in dem Verbindungskanal (3) untergebracht und dazu ausgebildet sind, mit den Sensormitteln (7) zusammenzuwirken, wobei der Verbindungskanal (3) zumindest teilweise von den Übertragungsmitteln (10) in Eingriff genommen wird, wobei die Übertragungsmittel (10) mindestens ein Druckübertragungselement (12) der elastisch verformbaren Art umfassen, das im Inneren des Verbindungskanals (3) so angeordnet ist, dass es mindestens die zweite Schnittstelle (5) abdeckt, und/oder mindestens ein Element (14) zur Übertragung der Temperatur des Blutes zum Temperatursensor (13),
**dadurch gekennzeichnet, dass** sie umfasst
- mindestens ein Element zum Stützen (8) der Sensormittel (7), das dem Verbindungselement (2) zugeordnet ist, um die zweite Schnittstelle (5) zu verschließen, wobei das Verbindungselement (2) mit dem Element zum Stützen (8) in dem Bereich verriegelt ist, in dem die Sensormittel (7) definiert sind, wobei die Sensormittel (7) an der Stelle angeordnet sind, an der sich die zweite Schnittstelle (5) befindet, wodurch eine Verbindung mit dem Verbindungskanal (3) entsteht.

2. Vorrichtung (1) nach Anspruch 1, **dadurch gekennzeichnet, dass** das Verbindungselement (2) einen an der ersten Schnittstelle (4) definierten Verbinder des Luer-Lock-Typs umfasst.

3. Vorrichtung (1) nach Anspruch 2, **dadurch gekennzeichnet, dass** das Verbindungselement (2) Mittel zur Verriegelung (6) mit der biomedizinischen Vorrichtung des Typs Drehkragen umfasst.

4. Vorrichtung (1) nach einem oder mehreren der vorhergehenden Ansprüche, **dadurch gekennzeichnet, dass** der Drucksensor (11) vom Typ Druckwandler ist.

5. Vorrichtung (1) nach einem oder mehreren der vorhergehenden Ansprüche, **dadurch gekennzeichnet, dass** die Übertragungsmittel (10) vom Typ biokompatibles Gel sind.

6. Vorrichtung (1) nach einem oder mehreren der vorhergehenden Ansprüche, **dadurch gekennzeichnet, dass** das Element zum Stützen (8) mindestens eine integrierte elektrische Schaltung (9) umfasst, die mit den Sensormitteln (7) in Wirkverbindung steht, wobei die elektrische Schaltung (9) dazu ausgebildet ist, die von den Sensormitteln (7) erzeugten elektrischen Impulse an eine Verarbeitungseinheit zu übertragen.

7. Vorrichtung (1) nach einem oder mehreren der vorhergehenden Ansprüche, **dadurch gekennzeichnet, dass** sie Mittel zur Aufnahme (15) des Elements zum Stützen (8) umfasst.

8. Vorrichtung (1) nach einem oder mehreren der vorhergehenden Ansprüche, **dadurch gekennzeichnet, dass** sie Mittel zur Verbindung (16) der elektrischen Schaltung (9) mit einer Einheit zur Verarbeitung der erfassten physikalischen Größen umfasst.

9. Vorrichtung (1) nach Anspruch 8, **dadurch gekennzeichnet, dass** die Mittel zur Verbindung (16) der elektrischen Schaltung (9) lösbar zugeordnet sind.

## Revendications

1. - Dispositif de raccord biomédical pour la mesure de la pression et/ou de la température du sang (1), comprenant :
- un élément de raccord tubulaire (2) comportant au moins un canal de liaison (3) définissant un premier orifice (4), pouvant être relié à un dispositif biomédical pour le traitement du sang, et un second orifice (5) opposé audit premier orifice (4) ;
- des moyens de détection (7), positionnés de manière adjacente audit second orifice (5) et communiquant avec ledit canal de liaison (3), aptes à détecter la pression et/ou la température du sang, lesdits moyens de détection (7) comprenant au moins un capteur de pression (11) apte à détecter la pression du sang et/ou au moins un capteur de température (13) apte à détecter la température du sang ;
- des moyens de transmission (10) pour transmettre ladite pression et/ou ladite température du sang auxdits moyens de détection (7), lesdits moyens de transmission (10) étant reçus dans ledit canal de liaison (3) et étant aptes à interagir avec lesdits moyens de détection (7), ledit canal de liaison (3) étant engagé au moins partiellement par lesdits moyens de transmission (10), lesdits moyens de transmission (10) comprenant au moins un élément de transmission de pression (12) du type élastiquement déformable et disposé à l'intérieur dudit canal de liaison (3) de façon à recouvrir au moins ledit second orifice (5) et/ou au moins un élément de transmission de température (14) du sang audit capteur de température (13),
**caractérisé par le fait qu'**il comprend :
- au moins un élément de support (8) desdits moyens de détection (7) associé audit élément de raccord (2), pour fermer ledit second orifice (5), ledit élément de raccord (2) étant verrouillé conjointement avec ledit élément de support (8) à la zone où sont définis lesdits moyens de détection (7), lesdits moyens de détection (7) étant disposés à l'endroit où se trouve ledit second orifice (5), ce qui entraîne une communication avec ledit canal de liaison (3).

2. - Dispositif (1) selon la revendication 1, **caractérisé par le fait que** ledit élément de raccord (2) comprend un connecteur du type Luer-Lock défini audit premier orifice (4).

3. - Dispositif (1) selon la revendication 2, **caractérisé par le fait que** ledit élément de raccord (2) comprend des moyens de verrouillage (6) dudit dispositif biomédical, du type d'un collier rotatif.

4. - Dispositif (1) selon l'une ou plusieurs des revendications précédentes, **caractérisé par le fait que** ledit capteur de pression (11) est du type d'un transducteur de pression.

5. - Dispositif (1) selon l'une ou plusieurs des revendications précédentes, **caractérisé par le fait que** lesdits moyens de transmission (10) sont du type d'un gel biocompatible.

6. - Dispositif (1) selon l'une ou plusieurs des revendications précédentes, **caractérisé par le fait que** ledit élément de support (8) comprend au moins un circuit électrique intégré (9), relié fonctionnellement auxdits moyens de détection (7), ledit circuit électrique (9) étant apte à transférer les impulsions électriques générées par lesdits moyens de détection (7) vers une unité de traitement.

7. - Dispositif (1) selon l'une ou plusieurs des revendications précédentes, **caractérisé par le fait qu'**il comporte des moyens de confinement (15) dudit élément de support (8).

8. - Dispositif (1) selon l'une ou plusieurs des revendications précédentes, **caractérisé par le fait qu'**il comporte des moyens de connexion (16) dudit circuit électrique (9) à une unité de traitement des grandeurs physiques détectées.

9. - Dispositif (1) selon la revendication 8, **caractérisé par le fait que** lesdits moyens de connexion (16) sont associés d'une manière amovible audit circuit électrique (9).
